# EUROPÄISCHE PATENTSCHRIFT

(11) **EP 0 927 222 B1**
(45) Veröffentlichungstag und Bekanntmachung des Hinweises auf die Patenterteilung: **16.05.2001**
(21) Anmeldenummer: 97943844.7
(22) Anmeldetag: 08.09.1997
(51) Int. Cl.: C08K 5/09, A61L 27/00

(54) **WIRKSTOFFHALTIGE THERMOPLASTISCHE POLYURETHANE**
THERMOPLASTIC POLYURETHANES CONTAINING ACTIVE SUBSTANCES
POLYURETHANNES THERMOPLASTIQUES CONTENANT DES PRINCIPES ACTIFS

(30) Priorität: 20.09.1996 DE 19638570
(43) Veröffentlichungstag der Anmeldung: 07.07.1999
(73) Patentinhaber: BAYER AG, 51368 Leverkusen (DE)
(72) Erfinder: PUDLEINER, Heinz, D-47800 Krefeld (DE); DUJARDIN, Ralf, D-47877 Willich (DE); HOBLER, Hartwin, D-42111 Wuppertal (DE)
(86) Internationale Anmeldenummer: EP9704868
(87) Internationale Veröffentlichungsnummer: WO9811860

(56) Entgegenhaltungen:
- EP-A- 0 659 442
- DE-A- 4 221 665
- US-A- 4 925 668
- US-A- 4 973 320

## Beschreibung

Die Erfindung betrifft pharmazeutische Wirkstoffe in homogener Verteilung enthaltende thermoplastische Polyurethane (TPU), Verfahren zu ihrer Herstellung und deren Verwendung in medizinischen Artikeln. Bei der Herstellung der TPU werden der Monomermischung pharmakologisch wirksame Verbindungen zugegeben.

In der Medizin verwendete Materialien, die in direktem Kontakt mit dem Blut stehen, z.B. künstliche Organe, künstliche Blutgefäße, Katheter, Bluttransfusionsvorrichtungen, müssen neben geeigneten mechanischen Eigenschaften und guter Biokompatibilität bzw. Blutverträglichkeit auch noch antithrombotische und/oder antibakterielle Wirkung aufweisen.

Polymermaterialien für medizinische Anwendungen, die Beschichtungen aufweisen, in denen Heparin oder ein anderer antithrombotisch wirksamer Stoff enthalten ist, sind bekannt. Da jedoch nur geringe Mengen an Wirkstoff in die aufgetragene Beschichtung eingebracht werden können, steht nicht für die gesamte Anwendungsdauer eine genügend hohe Konzentration des Wirkstoffs an der Oberfläche des medizinischen Artikels zur Verfügung.

Es sind auch Verfahren bekannt, antithrombotisch wirksame Substanzen in die äußere Polymerschicht medizinischer Artikel einzubringen. Nach EP-A 550 875 werden medizinische Artikel aus polymerem Material mit einem Lösungsmittel vorbehandelt, damit eine pharmazeutisch wirksame Schicht in das Polymere eindringen kann. Dieses Verfahren ist technisch aufwendig und verwendet organische Lösungsmittel, die meist nicht restlos aus dem Material entfernt werden können; zudem wird nur eine geringe Beladung der Oberfläche mit der Wirksubstanz erzielt.

Die antithrombotische Wirkung von Acetylsalicylsäure ist bekannt (Münchner Medizinische Wochenschrift 130 (1988) 809). Nach DE-C 42 21 665 werden Polymeren als Additive Ester bzw. Mischester der Acetylsalicylsäure zugesetzt, um die Blutgerinnung zu unterdrücken. Es hat sich jedoch als nachteilig erwiesen, daß nur spezielle Derivate der Acetylsalicylsäure eingesetzt werden können und die Oberflächen erst nach einiger Zeit ihre optimale Biokompatibilität erreichen, da der Wirkstoff erst an die Oberfläche des Polymeren wandern muß.

Nach EP-A 659 442 wird zur Beschleunigung dieses Migrationsprozesses vor der thermoplastischen Verarbeitung ein Gemisch aus Amidwachs und Acetylsäure-Ethylendiamin an das Polymergranulat angelagert. Das Amidwachs schmilzt bei der Verarbeitung und wandert aufgrund seiner Unverträglichkeit mit dem Polymeren an die Oberfläche des gefertigten Teils. Dadurch wird die Migration des Acetylsalicylsäure-derivats unterstützt. Es können jedoch nur begrenzte Mengen an Wirkstoff an das Granulat angelagert und dadurch in das medizinische Arbeitsmittel eingebracht werden. Darüber hinaus wird auch für dieses Verfahren ein spezielles Derivat der Acetylsalicylsäure benötigt.

In DE-C 4 221 665 werden medizinische Arbeitsmittel beschrieben, die Acetylsalicylsäureester als Zuschlagstoff enthalten. Dieser Zuschlagstoff wird vor der eigentlichen Formgebung dem hergestellten Kunststoff zugemischt.

Allen erwähnten Verfahren ist gemeinsam, dass das Ausrüsten des medizinischen Arbeitsmittels mit einer pharmakologisch wirksamen Substanz einen zusätzlichen Arbeitsschritt erforderlich macht, nämlich entweder eine Vorbehandlung des Polymermaterials vor der Verarbeitung oder eine Nachbehandlung der hergestellten Formkörper. Dies verursacht zusätzliche Kosten und bringt einen erhöhten Zeitaufwand bei der Produktion mit sich. Es wurde nun gefunden, dass man bei der Herstellung medizinischer Arbeitsmittel aus thermoplastischen Polyurethanen Wirkstoffe schon bei der Polymerherstellung zusetzen kann. Das erhaltene Polymer kann dann ohne zusätzlichen Aufwand in konventioneller Weise zu wirkstoffhaltigen medizinischen Arbeitsmitteln weiterverarbeitet werden

In US 4 973 320 wird ein Verfahren zur Herstellung von medizinischen Artikeln aus thermoplastischen Polyurethanen beschrieben, bei dem das oligodynamische Mittel dem Polyol vor Zugabe des Isocyanates zugemischt wird. Das Gemisch reagiert zum thermoplastischen Polyurethan, welches anschließend zum entsprechenden Formkörper verarbeitet wird.

Gegenstand der Erfindung ist somit ein Verfahren zur Herstellung wirkstoffhaltiger thermoplastisch verarbeitbarer Polyurethane durch Umsetzung der polyurethanbildenden Komponenten
A) organisches Diisocyanat,
B) lineares hydroxylterminiertes Polyol mit einem Molekulargewicht von 500 bis 10000,
C) Kettenverlängerer mit einem Molekulargewicht von 60 bis 500,
D) 0,01 bis 10 Gew.-%, bezogen auf die Gesamtmenge an Edukten, eines pharmakologisch wirksamen Stoffes ausgenommen oligodynamische Mittel,
wobei das Molverhältnis der NCO-Gruppen in A) zu den gegenüber Isocyanat reaktiven Gruppen in B) u. C) 0,9 bis 1,2 beträgt.

Als organische Diisocyanate A) kommen beispielsweise aliphatische, cycloaliphatische, araliphatische, heterocyclische und aromatische Diisocyanate in Betracht, wie sie in Justus Liebigs Annalen der Chemie, 562, S.75-136 beschrieben werden. Bevorzugt sind aliphatische und cycloaliphatische Diisocyanate.

Im einzelnen seien beispielhaft genannt: aliphatische Diisocyanate, wie Hexamethylendiisocyanat, cycloaliphatische Diisocyanate, wie Isophorondiisocyanat, 1,4-Cyclohexan-diisocyanat, 1-Methyl-2,4-cyclohexan-diisocyanat und 1-Methyl-2,6-cyclohexan-diisocyanat sowie die entsprechenden Isomerengemische, 4,4'-Dicyclohexylmethan-diisocyanat, 2,4'-Dicyclohexylmethan-diisocyanat und 2,2'-Dicyclohexylmethan-diisocyanat sowie die entsprechenden Isomerengemische, aromatische Diisocyanate, wie 2,4-Toluylendiisocyanat, Gemische aus 2,4-Toluylendiisocyanat und 2,6-Toluylendiisocyanat, 4,4'-Diphenylmethandiisocyanat, 2,4'-Diphenylmethandiisocyanat und 2,2'-Diphenylmethandiisocyanat, Gemische aus 2,4'-Diphenylmethandiisocyanat und 4,4'-Diphenylmethandiisocyanat, urethanmodifizierte flüssige 4,4'-Diphenylmethandiisocyanate und 2,4'-Diphenylmethandiisocyanate, 4,4'-Diisocyanatodiphenyl-ethan-(1,2) und 1,5-Naphthylendiisocyanat. Vorzugsweise verwendet werden 1,6-Hexamethylendiisocyanat, Isophorondiisocyanat, Dicyclohexylmethandiisocyanat, Diphenylmethandiisocyanat-Isomerengemische mit einem 4,4'-Diphenylmethandiisocyanatgehalt von >96 Gew.-% und insbesondere 4,4'-Diphenylmethandiisocyanat und 1,5-Naphthylendiisocyanat. Die genannten Diisocyanate können einzeln oder in Form von Mischungen untereinander zur Anwendung kommen. Sie können auch zusammen mit bis zu 15 Gew.-% (berechnet auf die Gesamtmenge an Diisocyanat) eines Polyisocyanates verwendet werden, beispielsweise Triphenylmethan-4,4',4"-triisocyanat oder Polyphenyl-polymethylen-polyisocyanaten.

Als Komponente B) werden lineare hydroxylterminierte Polyole mit einem mittleren Molekulargewicht Mₙ von 500 bis 10000, bevorzugt 500 bis 5000, besonders bevorzugt 600 bis 2000 eingesetzt. Produktionsbedingt enthalten diese oft kleine Mengen an nichtlinearen Verbindungen. Häufig spricht man daher auch von "im wesentlichen linearen Polyolen". Bevorzugt sind Polyester-, Polyether-, Polycarbonat-Diole, hydroxylterminierte Polybutadiene, hydroxylterminierte Polysiloxane oder Gemische aus diesen.

Geeignete Polyether-Diole können dadurch hergestellt werden, daß man ein oder mehrere Alkylenoxide mit 2 bis 4 Kohlenstoffatomen im Alkylenrest mit einem Startermolekül, das zwei aktive Wasserstoffatome gebunden enthält, umsetzt. Als Alkylenoxide seien z.B. genannt: Ethylenoxid, 1,2-Propylenoxid, Epichlorhydrin und 1,2-Butylenoxid und 2,3-Butylenoxid. Vorzugsweise werden Ethylenoxid, Propylenoxid und Mischungen aus 1,2-Propylenoxid und Ethylenoxid eingesetzt. Die Alkylenoxide können einzeln, alternierend nacheinander oder als Mischungen verwendet werden. Als Startermoleküle kommen beispielsweise in Betracht: Wasser, Aminoalkohole, wie N-Alkyl-diethanolamine, beispielsweise N-Methyl-diethanol-amin, und Diole, wie Ethylenglykol, 1,3-Propylenglykol, 1,4-Butandiol und 1,6-Hexandiol. Gegebenenfalls können auch Mischungen von Startermolekülen eingesetzt werden. Geeignete Polyether-Diole sind ferner die hydroxylgruppenhaltigen Polymerisationsprodukte des Tetrahydrofurans. Es können auch trifunktionelle Polyether in Anteilen von 0 bis 30 Gew.-%, bezogen auf die bifunktionellen Polyether, eingesetzt werden, jedoch höchstens in solcher Menge, daß ein thermoplastisch verarbeitbares Produkt entsteht. Die im wesentlichen linearen Polyether-Diole können sowohl einzeln als auch in Form von Mischungen untereinander zur Anwendung kommen.

Geeignete Polyester-Diole können beispielsweise aus Dicarbonsäuren mit 2 bis 12 Kohlenstoffatomen, vorzugsweise 4 bis 6 Kohlenstoffatomen, und mehrwertigen Alkoholen hergestellt werden. Als Dicarbonsäuren kommen beispielsweise in Betracht: aliphatische Dicarbonsäuren, wie Bernsteinsäure, Glutarsäure, Adipinsäure, Korksäure, Azelainsäure und Sebacinsäure und aromatische Dicarbonsäuren, wie Phthalsäure, Isophthalsäure und Terephthalsäure. Die Dicarbonsäuren können einzeln oder als Gemische, z.B. in Form einer Bernstein-, Glutar- und Adipinsäuremischung, verwendet werden. Zur Herstellung der Polyester-Diole kann es gegebenenfalls vorteilhaft sein, anstelle der Dicarbonsäuren die entsprechenden Dicarbonsäurederivate, wie Carbonsäurediester mit 1 bis 4 Kohlenstoffatomen im Alkoholrest, Carbonsäureanhydride oder Carbonsäurechloride zu verwenden. Beispiele für mehrwertige Alkohole sind Glykole mit 2 bis 10, vorzugsweise 2 bis 6 Kohlenstoffatomen, wie Ethylenglykol, Diethylenglykol, 1,4-Butandiol, 1,5-Pentandiol, 1,6-Hexandiol, 1,10-Decandiol, 2,2-Dimethyl-1,3-propandiol, 1,3-Propandiol und Dipropylenglykol. Je nach den gewünschten Eigenschaften können die mehrwertigen Alkohole allein oder gegebenenfalls in Mischung untereinander verwendet werden. Geeignet sind ferner Ester der Kohlensäure mit den genannten Diolen, insbesondere solchen mit 4 bis 6 Kohlenstoffatomen, wie 1,4-Butandiol oder 1,6-Hexandiol, Kondensationsprodukte von Hydroxycarbonsäuren, beispielsweise Hydroxycapronsäure und Polymerisationsprodukte von Lactonen, beispielsweise gegebenenfalls substituierten Caprolactonen Als Polyester-Diole vorzugsweise verwendet werden Ethandiol-polyadipate, 1,4-Butandiol-polyadipate, Ethandiol-1,4-butandiol-polyadipate, 1,6-Hexandiol-neopentylglykol-polyadipate, 1,6-Hexandiol-1,4-butandiol-polyadipate und Poly-caprolactone. Die Polyester-Diole können einzeln oder in Form von Mischungen untereinander zur Anwendung kommen.

Als Kettenverlängerungsmittel C) werden Diole, Diamine oder Aminoalkohole mit einem Molekulargewicht von 60 bis 500 eingesetzt, vorzugsweise aliphatische Diole mit 2 bis 14 Kohlenstoffatomen, wie z.B. Ethandiol, 1,6-Hexandiol, Diethylenglykol, Dipropylenglykol und insbesondere 1,4-Butandiol. Geeignet sind jedoch auch Diester der Terephthalsäure mit Glykolen mit 2 bis 4 Kohlenstoffatomen, wie z.B. Terephthalsäure-bis-ethylenglykol oder Terephthalsäure-bis-1,4-butandiol, Hydroxyalkylenether des Hydrochinons, wie z.B. 1,4-Di(-hydroxyethyl)hydrochinon, ethoxylierte Bisphenole, (cyclo)aliphatische Diamine, wie z.B. Isophorondiamin, Ethylendiamin, 1,2-Propylen-diamin, 1,3-Propylen-diamin, N-Methyl-propylen-1,3-diamin, 1,6-Hexamethylendiamin, 1,4-Diaminocyclohexan, 1,3-Diaminocyclohexan, N,N'-Dimethyl-ethylendiamin und 4,4'-Dicyclohexylmethandiamin und aromatische Diamine, wie z.B. 2,4-Toluylen-diamin und 2,6-Toluylen-diamin, 3,5-Diethyl-2,4-toluylen-diamin und 3,5-Diethyl-2,6-toluylen-diamin und primäre mono-, di-, tri- oder tetraalkylsubstituierte 4,4'-Diaminodiphenylmethane oder Aminoalkohole wie Ethanolamin, 1-Aminopropanol, 2-Aminopropanol. Es können auch Gemische der oben genannten Kettenverlängerer eingesetzt werden. Daneben können auch kleinere Mengen an tri- oder höherfunktionellen Vemetzern zugesetzt werden, z.B. Glycerin, Trimethylolpropan, Pentaerythrit, Sorbit.

Weiterhin können in geringen Mengen auch übliche monofunktionelle Verbindungen eingesetzt werden, z.B. als Kettenabbrecher oder Entformungshilfen. Beispielhaft genannt seien Alkohole wie Oktanol und Stearylalkohol oder Amine wie Butylamin und Stearylamin.

Als Wirkstoffe D) kann eine Vielzahl von pharmakologisch wirksamen Substanzen eingesetzt werden, beispielsweise antithrombotisch oder antibiotisch wirksame Stoffe. Beispiele für erfindungsgemäß verwendbare antithrombotisch wirksame Substanzen sind Acetylsalicylsäure und deren Alkali- und Erdalkalisalze sowie Derivate des Dihydropyridins wie N-methyl-2,6-dimethyl-3,5-di(methoxycarbonyl)-4-(4-trifluormethylphenyl)-1,4-dihydropyridin (I).

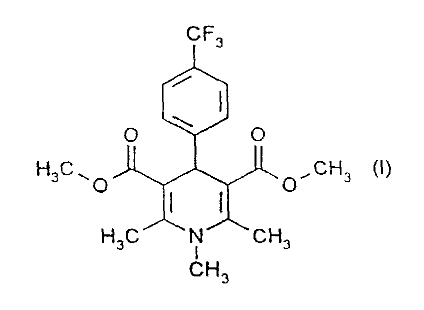

Beispiele für antibiotisch wirksame Substanzen sind Ciprofloxacin und Fosfomycin. Die Wirkstoffe D) werden in Mengen von 0,01 bis 10 Gew.-%, bevorzugt 0,05 bis 7 Gew.-%, besonders bevorzugt 0,1 bis 5 Gew.-%, bezogen auf die Gesamtmenge an Edukten, eingesetzt. Da die Wirkstoffe bereits bei der Herstellung des TPU der Reaktionsmischung zugesetzt werden, wird eine homogene Verteilung der pharmakologisch wirksamen Substanzen im Endprodukt erzielt, ohne daß zusätzliche Arbeitsschritte erforderlich wären.

Empfindliche Wirkstoffe wie Heparin sind bei den für die thermoplastische Verarbeitung von Polymeren erforderlichen hohen Temperaturen von etwa 150°C bis 210°C normalerweise nicht stabil. Es hat sich überraschend gezeigt, daß durch das erfindungsgemäße Verfahren solche Wirkstoffe durch Einbindung in die Polymermatrix soweit stabilisiert werden, daß auch nach thermoplastischer Verarbeitung ihre Wirksamkeit erhalten bleibt.

Der Zusatz antithrombotisch wirksamer Substanzen in TPU verbessert die Blutverträglichkeit der aus diesem Material hergestellten Artikel, ein Zusatz von antibiotisch wirksamen Stoffen wirkt einer Ansiedlung von Keimen auf der Oberfläche entgegen. Der eingebaute Wirkstoff kann im Laufe der Zeit zwar teilweise an die Oberfläche der medizinischen Artikel diffundieren, er wird aber nicht in therapeutisch wirksamen Konzentrationen an das umgebende Medium, z.B. Gewebe, Blut oder andere Körperflüssigkeiten, abgegeben. Tritt durch physiologische Prozesse ein Abbau der Wirksubstanzen an der Grenzfläche zwischen Polymer und umgebendem Medium auf, so kann dieser durch den Diffusionsprozeß kompensiert werden. Dadurch ist für einen ständigen Nachschub an Wirksubstanz gesorgt, so daß der gewünschte Schutz der Oberfläche des medizinischen Artikels auch über einen längeren Zeitraum gewährleistet ist.

Die molaren Verhältnisse der Aufbaukomponenten können über einen breiten Bereich variiert werden, woduch sich die Eigenschaften des Produkts einstellen lassen. Bewährt haben sich molare Verhältnisse von Polyolen zu Kettenverlängerern von 1:1 bis 1:12. Das Molverhältnis von Diisocyanaten und Polyolen beträgt bevorzugt 1,2:1 bis 30:1. Besonders bevorzugt sind Verhältnisse von 2:1 bis 12:1. Zur Herstellung der TPU können die Aufbaukomponenten, gegebenenfalls in Gegenwart von Katalysatoren, Hilfsmitteln und Zusatzstoffen, in solchen Mengen zur Reaktion gebracht werden, daß das Äquivalenzverhältnis von NCO-Gruppen zur Summe der NCO-reaktiven Gruppen, insbesondere der Hydroxy- oder Aminogruppen der niedermolekularen Diole/Triole, Amine und der Polyole 0,9:1 bis 1,2:1, vorzugsweise 0,98:1 bis 1,05:1, besonders bevorzugt 1,005:1 bis 1,01:1 beträgt.

Die erfindungsgemäßen Polyurethane können ohne Katalysatoren hergestellt werden; in manchen Fällen kann der Einsatz von Katalysatoren jedoch angezeigt sein. Im allgemeinen werden die Katalysatoren in Mengen von bis zu 100 ppm, bezogen auf die Gesamtmenge an Edukten, verwendet. Geeignete erfindungsgemäße Katalysatoren sind die nach dem Stand der Technik bekannten und üblichen tertiären Amine, wie z.B. Triethylamin, Dimethylcyclohexylamin, N-Methylmorpholin, N,N'-Dimethyl-piperazin, 2-(Dimethylamino-ethoxy)-ethanol, Diazabicyclo-(2,2,2)-octan und ähnliche sowie insbesondere organische Metallverbindungen wie Titansäureester, Eisenverbindungen, Zinnverbindungen, z.B. Zinndiacetat, Zinndioctoat, Zinndilaurat oder die Zinndialkylsalze aliphatischer Carbonsäuren. Bevorzugt sind Dibutylzinndiacetat und Dibutylzinndilaurat; von diesen genügen Mengen von 1 bis 10 ppm, um die Reaktion zu katalysieren.

Neben den TPU-Komponenten und den Katalysatoren können auch andere Hilfsmittel und Zusatzstoffe zugesetzt werden. Genannt seien beispielsweise Gleitmittel wie Fettsäureester, deren Metallseifen, Fettsäureamide und Siliconverbindungen, Antiblockmittel, Inhibitoren, Stabilisatoren gegen Hydrolyse, Licht, Hitze und Verfärbung, Flammschutzmittel, Farbstoffe, Pigmente, anorganische oder organische Füllstoffe und Verstärkungsmittel. Verstärkungsmittel sind insbesondere faserartige Verstärkungsstoffe wie anorganische Fasern, die nach dem Stand der Technik hergestellt werden und auch mit einer Schlichte beaufschlagt sein können. Nähere Angaben über die genannten Hilfs- und Zusatzstoffe sind der Fachliteratur zu entnehmen, beispielsweise J.H. Saunders, K.C. Frisch: "High Polymers", Band XVI, Polyurethane, Teil 1 und 2, Interscience Publishers 1962 bzw. 1964, R.Gächter, H.Müller (Ed.): Taschenbuch der Kunststoff-Additive, 3.Ausgabe, Hanser Verlag, München 1989, oder DE-A 29 01 774.

Der Aufbau der thermoplastisch verarbeitbaren Polyurethanelastomeren erfolgt bevorzugt schrittweise im sogenannten Prepolymerverfahren. Beim Prepolymerverfahren wird aus dem Polyol und dem Diisocyanat ein isocyanathaltiges Prepolymer gebildet, das in einem zweiten Schritt mit dem Kettenverlängerer umgesetzt wird. Die TPU können kontinuierlich oder diskontinuierlich hergestellt werden. Die bekanntesten technischen Herstellverfahren sind das Bandverfahren und das Extruderverfahren.

Gegenstand der Erfindung ist auch die Verwendung der erfindungsgemäß behandelten thermoplastischen Polyurethane zur Herstellung medizinischer Artikel, z.B. von Kathetern, Schläuchen, Blutbeuteln, Folien oder Formkörpern für Implantate.

Die nach dem erfindungsgemäßen Verfahren erhaltenen TPU können nach üblichen Verfahren wie Extrusion oder Spritzgießen zu Formkörpern, Schläuchen oder Folien verarbeitet werden. Diese sind stippenfrei, flexibel, kleben nicht und können problemlos nach den gängigen Verfahren sterilisiert werden.

### Beispiele

### Beispiel 1

In einem 61-Planschliffkolben mit Rührer, Thermometer und Rückflußkühler wurden 2880 g (1,373 mol) Polytetrahydrofuran mit einem mittleren Molekulargewicht von 2000 g/mol vorgelegt und bei 120°C/14 mbar 1 Stunde lang getrocknet. Dann wurden 0,12 g Dibutylzinnlaurat als Katalysator und 1528,9 g (5,829 mol) H₁₂MDI zugegeben und es wurde bei 120°C bis zum Erreichen eines NCO-Wertes von 8,5 Gew.-% gerührt. In dieses Prepolymer wurden zunächst 48,8 g (1,0 Gew.-%, bezogen auf die gesamte Eduktmenge) Calcium-Acetylsalicylat und anschließend als Kettenverlängerer 391,1 g (4,341 mol) 1,4-Butandiol eingerührt. Nach ca. 60 Sekunden wurde die Reaktionsmischung auf ein beschichtetes Blech gegossen, wo sie nach wenigen Minuten erstarrte. Zur Vervollständigung der Reaktion wurde 12 Stunden lang bei 100°C nachgetempert.

Die ausreagierten Platten wurden zerschnitten und zerkleinert. Das Häckselgranulat wurde auf einem Zweiwellen-Brabender-Meßextruder extrudiert und stranggranuliert. Für die Blutverträglichkeitstests wurden aus dem Zylindergranulat Schläuche mit 3 mm Innendurchmesser extrudiert.

### Beispiel 2

In einem 61-Planschliffkolben mit Rührer, Thermometer und Rückflußkühler wurden 2880 g (1,373 mol) Polytetrahydrofuran mit einem mittleren Molekulargewicht von 2000 g/mol vorgelegt und bei 120°C/14 mbar 1 Stunde lang getrocknet. Dann wurden 0,12 g Dibutylzinnlaurat als Katalysator und 1528,9 g (5,829 mol) H₁₂MDI zugegeben und es wurde bei 120°C bis zum Erreichen eines NCO-Wertes von 8,5 Gew.-% gerührt. In dieses Prepolymer wurden zunächst 4,8 g (0,1 Gew.-%, bezogen auf die gesamte Eduktmenge) N-methyl-2,6-dimethyl-3,5-di(methoxycarbonyl)-4-(4-trifluormethylphenyl)-1,4-dihydropyridin und anschließend als Kettenverlängerer 391,1 g (4,341 mol) 1,4-Butandiol eingerührt. Nach ca. 60 Sekunden wurde die Reaktionsmischung auf ein beschichtetes Blech gegossen, wo sie nach wenigen Minuten erstarrte. Zur Vervollständigung der Reaktion wurde 12 Stunden lang bei 100°C nachgetempert.

Die ausreagierten Platten wurden zerschnitten und zerkleinert. Das Häckselgranulat wurde auf einem Zweiwellen-Brabender-Meßextruder extrudiert und stranggranuliert. Für die Blutverträglichkeitstests wurden aus dem Zylindergranulat Schläuche mit 3 mm Innendurchmesser extrudiert.

### Beispiel 3 (Vergleich)

In einem 61-Planschliffkolben mit Rührer, Thermometer und Rückflußkühler wurden 2880 g (1,373 mol) Polytetrahydrofuran mit einem mittleren Molekulargewicht von 2000 g/mol vorgelegt und bei 120°C/14 mbar 1 Stunde lang getrocknet. Dann wurden 0,12 g Dibutylzinnlaurat als Katalysator und 1528,9 g (5,829 mol) H₁₂MDI zugegeben und es wurde bei 120°C bis zum Erreichen eines NCO-Wertes von 8,5 Gew.-% gerührt. In dieses Prepolymer wurden als Kettenverlängerer 391,1 g (4,341 mol) 1,4-Butandiol eingerührt Nach ca. 60 Sekunden wurde das Reaktionsgemisch auf ein beschichtetes Blech gegossen, wo es nach wenigen Minuten erstarrte. Zur Vervollständigung der Reaktion wurde 12 Stunden lang bei 100°C nachgetempert. Die ausreagierten Platten wurden zerschnitten und zerkleinert.

Ein Teil des Häckselgranulats wurde auf einem Zweiwellen-Brabender-Meßextruder extrudiert und stranggranuliert. Für die Blutverträglichkeitstests wurden aus dem Zylindergranulat Schläuche mit 3 mm Innendurchmesser extrudiert.

### Beispiel 4 (Vergleich)

Zur Herstellung eines Masterbatchs wurden 475,2 g des Häckselgranulats aus Beispiel 3 in Ethanol suspendiert und mit 4,8 g N-methyl-2,6-dimethyl-3,5-di(methoxycarbonyl)-4-(4-trifluormethylphenyl)-1,4-dihydropyridin versetzt. Die Suspension wurde am Rotationsverdampfer vom Ethanol befreit und im Trockenschrank bei 40°C am Vakuum getrocknet.

Der 1 Gew.-% Masterbatch wurde mit 4320 g wirkstofffreiem Häckselgranulat gemischt und auf einem Zweiwellen-Brabender-Meßextruder extrudiert. Es wurde eine klare, stippenfreie Schmelze erhalten, die nach Abkühlung im Wasserbad und Stranggranulation ein farbloses, klares und nicht klebriges Zylindergranulat ergab. Für die Blutverträglichkeitstests wurden aus dem Zylindergranulat Schläuche mit 3 mm Innendurchmesser extrudiert.

### Beispiel 5 (Vergleich)

Zur Herstellung eines Masterbatchs wurden 432 g des Häckselgranulats aus Beispiel 3 in Ethanol suspendiert und mit 48 g Calcium-Acetylsalicylat versetzt. Die Suspension wurde am Rotationsverdampfer vom Ethanol befreit und im Trockenschrank bei 40°C am Vakuum getrocknet.

Der 10 Gew.-% Masterbatch wurde mit 4320 g wirkstofffreiem Häckselgranulat gemischt und auf einem Zweiwellen-Brabender-Meßextruder extrudiert. Es wurde eine klare, stippenfreie Schmelze erhalten, die nach Abkühlung im Wasserbad und Stranggranulation ein farbloses, klares und nicht klebriges Zylindergranulat ergab. Für die Blutverträglichkeitstests wurden aus dem Zylindergranulat Schläuche mit 3 mm Innendurchmesser extrudiert.

### Dynamische in-vitro-Blutverträglichkeitstests

Für die Charakterisierung der in-vitro-Thrombogenität der Polymeroberflächen im Blutkontakt wurde frisch entnommenes menschliches Vollblut mit Citrat oder Hirudin versetzt, um die Gerinnung zu unterdrücken. Nachdem ein Schlauch aus dem zu prüfenden Material mit Blut gefüllt und die Schlauchenden verbunden worden waren, wurde der Schlauchring für eine vorgegebene Zeit auf eine vertikal mit konstanter Drehzahl rotierende Trommel gespannt.

Zur Beurteilung der Blutverträglichkeit wurden verschiedene Blutgerinnungsfaktoren aus unterschiedlichen Stadien der Gerinnungskaskade, Blutplättchen, sowie weiße und rote Blutkörperchen bestimmt und nach folgendem Score-Punktesystem bewertet: Plättchenaktivierung 0-10 Punkte, Plasmatische Gerinnung 0-30 Punkte, Hämolyse 0-10 Punkte, Proteolyse 0-10 Punkte. Maximal konnten 60 Punkte erreicht werden; je geringer die erzielte Punktezahl, desto besser die Blutverträglichkeit. Die Ergebnisse der Untersuchungen sind in Tabelle 1 zusammengefaßt.

**Tabelle 1:**

| In-vitro-Blutverträglichkeit | | | | | |
|---|---|---|---|---|---|
| | Beispiel 1 | Beispiel 2 | Beispiel 3* | Beispiel 4* | Beispiel 5* |
| Plättchenaktivierung | 3 | 3 | 2 | 3 | 2 |
| Plasmatische Gerinnung | 1 | 2 | 7 | 3 | 3 |
| Hämolyse | 5 | 1 | 4 | 0 | 0 |
| Proteolyse | 1 | 1 | 1 | 0 | 1 |
| Summe | 10 | 7 | 14 | 6 | 6 |

| | | | | | |
|---|---|---|---|---|---|
| * nicht erfindungsgemäße Vergleichsbeispiele | | | | | |

### Beispiel 6 (Vergleich)

In einem Planschliffkolben mit Rührer und Innenthermometer wurden 720 Gew.-Teile Polytetrahydrofuran mit einem mittleren Molekulargewicht von 2000 g/mol und 76,6 Gew.-Teile 1,6-Hexandiol vorgelegt und bei 120°C/14 mbar 1 Stunde lang getrocknet. Dann wurden 329,8 Gew.-Teile Isophorondiisocyanat zugegeben und es wurde 3 Stunden lang bei 120°C bis zum Erreichen eines NCO-Wertes von 3,5 Gew.-% gerührt. Anschließend wurden 12 Gew.-Teile Ethylenbisstearylamid und 15,3 Gew.-Teile Di-n-butylamin zugegeben. Dieses Prepolymer wurde in 634 Gew.-Teilen Toluol gelöst und unter Rühren bei Raumtemperatur einer Lösung von 73,6 Gew.-Teilen 5-Amino-3-aminomethyl-1,3,3-trimethylcyclohexan in 2990 Gew.-Teilen eines Gemischs aus Toluol und Isopropanol im Verhältnis 70:30 zugetropft. Es wurde eine farblose, transparente und homogene Lösung erhalten, die nach Trocknung einen farblosen, transparenten und nicht klebenden Film ergab. Dieser wurde gehäckselt und zu Prüfkörpern spritzgegossen, an denen die Bakterienadhasion in Suspensionen von S. aureus bzw. S. epidermis gemessen wurde.

### Beispiel 7

Ein analog Beispiel 6 hergestelltes Prepolymer wurde in 634 Gew.-Teilen Toluol gelöst und unter Rühren bei Raumtemperatur einer Lösung von 73,6 Gew.-Teilen 5-Amino-3-aminomethyl-1,3,3-trimethylcyclohexan und 12,4 Gew.-Teilen Ciprofloxacin in 2990 Gew.-Teilen eines Gemischs aus Toluol und Isopropanol im Verhältnis 70:30 zugetropft. Es wurde eine farblose, transparente und homogene Lösung erhalten, die nach Trocknung einen farblosen, transparenten und nicht klebenden Film ergab. Dieser wurde gehäckselt und zu Prüfkörpern spritzgegossen, an denen die Bakterienadhäsion in Suspensionen von S. aureus bzw. S. epidermis gemessen wurde. Im Vergleich zu der nach Beispiel 6 hergestellten wirkstofffreien Probe wurde eine Verringerung der Bakterienadhäsion um mehr als 99% festgestellt.

## Patentansprüche

1. Verfahren zur Herstellung wirkstoffhaltiger thermoplastisch verarbeitbarer Polyurethane durch Umsetzung der polyurethanbildenden Komponenten
A) organisches Diisocyanat,
B) lineares hydroxylterminiertes Polyol mit einem Molekulargewicht von 500 bis 10000,
C) Kettenverlängerer mit einem Molekulargewicht von 60 bis 500,
D) 0,01 bis 10 Gew.-%, bezogen auf die Gesamtmenge an Edukten, eines pharmakologisch wirksamen Stoffes ausgenommen oligodynamische Mittel,
wobei das Molverhältnis der NCO-Gruppen in A) zu den gegenüber Isocyanat reaktiven Gruppen in B) u. C) 0,9 bis 1,2 beträgt.

2. Verwendung der gemäß Anspruch 1 hergestellten wirkstoffhaltigen Polyurethane zur Herstellung medizinischer Artikel.

## Claims

1. A method of producing thermoplastically processable polyurethanes which contain active ingredients by the reaction of the polyurethane-forming components
A) an organic diisocyanate,
B) a linear hydroxyl-terminated polyol with a molecular weight of 500 to 10,000,
C) chain extenders with a molecular weight of 60 to 500, and
D) 0.01 to 10 % by weight, with respect to the total amount of starting materials, of a pharmacologically active substance, with the exception of oligodynamic agents,
wherein the molar ratio of the NCO groups in A) to the groups in B) and C) which are reactive towards isocyanate is 0.9 to 1.2.

2. The use of polyurethanes which contain active ingredients and which are produced according to claim 1 for the production of medical articles.

## Revendications

1. Procédé de préparation de polyuréthanes transformables de manière thermoplastique contenant des composés actifs par réaction des composants formant des polyuréthanes
A) un diisocyanate organique,
B) un polyol linéaire se terminant par un groupe hydroxyle avec une masse moléculaire de 500 à 10000,
C) un agent allongeant les chaînes avec une masse moléculaire de 60 à 500,
D) 0,01 à 10% en poids, rapporté à la quantité totale de composés de départ, d'un composé actif du point de vue pharmacologique à l'exception d'un agent oligodynamique,
dans lequel le rapport molaire des groupes NCO dans A) aux groupes dans B) et C) réactifs avec un isocyanate est 0,9 à 1,2.

2. Utilisation des polyuréthanes contenant des composés actifs préparés selon la revendication 1 pour la préparation d'articles médicaux.
